# EUROPÄISCHE PATENTSCHRIFT

(11) **EP 2 072 054 B1**
(45) Veröffentlichungstag und Bekanntmachung des Hinweises auf die Patenterteilung: **07.11.2018**
(21) Anmeldenummer: 07024973.5
(22) Anmeldetag: 21.12.2007
(51) Int. Cl.: A61K 36/16, A23L 33/105

(54) **Verwendung eines Extraktes aus Blättern von Ginkgo biloba**
Use of a ginkgo biloba leaf extract
Utilisation d'un extrait de feuilles de ginkgo biloba

(43) Veröffentlichungstag der Anmeldung: 24.06.2009
(60) Teilanmeldung: 18193631.1
(73) Patentinhaber: Dr. Willmar Schwabe GmbH & Co. KG, 76227 Karlsruhe (DE)
(72) Erfinder: Herrmann, Joachim, 68789 St. Leon-Rot (DE); Hörr, Robert, 76227 Karlsruhe (DE)
(74) Vertreter: Adam, Holger

(56) Entgegenhaltungen:
- EP-A- 1 559 430
- WO-A-02/13841
- WO-A-2006/009373
- WO-A-2006/117168
- WO-A-2006/117169
- WO-A-2006/117170
- WO-A-2006/117171
- ANDREY SCRIPNIKOV ET AL: "Effects of Ginkgo biloba Extract EGb 761 on neuropsychiatric symptoms of dementia: findings from a randomised controlled trial" WIENER MEDIZINISCHE WOCHENSCHRIFT, Bd. 157, Nr. 13-14, Juli 2007 (2007-07), Seiten 295-300, XP002480016
- MARTIEN VAN DONGEN ET AL: "Ginkgo for elderly people with dementia and age-associated memmory impairment: a randomized clinical trial" JOURNAL OF CLINICAL EPIDEMIOLOGY, Bd. 56, 2003, Seiten 367-376, XP002480015
- A. POPA: "Ginkgo Biloba and Memory", PHARMACOTHERAPY UPDATE, vol. v, no. v, September 2002 (2002-09), - October 2002 (2002-10),
- Phylis A. Balch: "Prescription for Herbal Healing", 2002, Avery, USA pages 183-186,
- S. KANOWSKI ET AL: "Gingko biloba Extract EGb 761 in Dementia: Intent to treat Analyses of a 24-week, Multi-center, Double-blind, Placebo-controlled, Randomized Trial", PHARMACOPSYCHIATRY, vol. 36, 2003, pages 297-303,
- R. IHL ET AL: "Efficacy and safety of a once-daily formulation of Ginkgo biloba extract EGb 761 in dementia with neuropsychiatric features. a randomized controlled trial", INTERNATIONAL JOURNAL OF GERIATRIC PSYCHIATRY, vol. 26, 2011, pages 1186-1194,
- H. HERRSCHAFT ET AL: "Ginkgo biloba extract EGb 761 in dementia with neuropsychiatric features: a randomized, placebo-controlled trial to confirm the efficacy and safety of a daily dose of 240 mg", JPOURNALOF PSYCHIATRIC RESEARCH, vol. 26, 2012, pages 716-723,
- B. GRASS-KAPANKE ET AL: "Effects of Ginkgo biloba special extract EGb 761 in very mild cognitive impairment (vMCI)", NEUROSCIENCE AND MEDICINE, vol. 2, 2011, pages 48-56,
- SI GAVRILOVA ET AL: "Efficy and safety of Ginkgo biloba extract EGb 761 in mild cognitive impairment with neuropsychiatric symptoms: A randomized, placebo-controlled, double-blind, multi-center trial", INTERNATIONAL JOURNAL OF GERIATRIC PSYCHIATRY, vol. 29, 2014, pages 1087-1095,
- "Pschyrembel Klinisches Wörterbuch", 1990, Walter de Gruyter page 335,
- JESSEN ET AL: "Prediction of dementia by subjective memory impairment", ARCHIVES OF GENERAL PSYCHIATRY, vol. 67, 2010, pages 414-422,
- REISBERG ET AL: "Outcome over sven years of healthy adults with and without subjective cognitive impairment", ALZEHEIMER'S AND DEMENTIA, vol. 6, 2010, pages 11-24,
- BARNES ET AL: "Memory complaints are related to Alzheimer disease pathology in older persons", NEUROLOGY, vol. 67, 2009, pages 1581-1885,
- European Medicines Agency, European Union herbal monograph on Ginkgo biloba L.folium
- Chapter 1, Marketing Authorisation: In: "Notice to Applicants,Procedures for marketing authorisation", November 2015 (2015-11), European Commission vol. 2A
- , Retrieved from the Internet: URL:http://www.clinicaltrialsregister.eu/c tr-search/trial/2005-003747-31/LV [retrieved on 2016]
- , Retrieved from the Internet: URL:www.clinicaltrialsregister.eu/ctr-sear ch/trial/2005-005765-11/EE [retrieved on 2016]
- S. GAUTHIER ET AL: CLINICAL INVESTIGATIONS IN AGING, vol. 9, 2014, pages 2065-2077,
- O. NAPRYEYENKO ET AL: DRUG RESEARCH, vol. 57, no. 1, 2007, pages 4-11,
- L. S. SCHNEIDER ET AL.: CURRENT ALZHEIMER RESEARCH, vol. 2, 2005, pages 541-551,
- T. M. ITIL ET AL: AMERICAN JOURNAL OF THERAPEUTICS, vol. 3, 1996, pages 63-73,

## Beschreibung

Die vorliegende Erfindung betrifft einen Extrakt aus Blättern von Ginkgo biloba als Mittel zur Verwendung zur Behandlung und Vorbeugung des dementiellen Syndroms sowie dessen Frühformen und Vorstufen, wobei einmal täglich 220 bis 260 mg Ginkgo-Extrakt verabreicht werden.

Extrakte aus den Blättern von Ginkgo biloba (Ginkgo-Extrakte) werden seit Jahrzehnten als Arzneimittel verwendet. Derzeit werden sie zur Behandlung verschiedener Arten des dementiellen Syndroms und dessen Symptomen sowie von cerebralen und peripheren Durchblutungsstörungen verwendet. Inhaltsstoffe, mit denen die Wirksamkeit verknüpft ist, sind Terpenlaktone (Ginkgolide A, B, C und Bilobalid) sowie Glycoside von Flavonen (Quercetin, Kämpferol und Isorhamnetin).

Die meisten arzneilich verwendeten Ginkgo-Extrakte sind standardisiert auf 22,0 bis 27,0 Gew.-% Flavonglycoside, 5,0 bis 7,0 Gew.-% Terpenlactone und höchstens 5 ppm Ginkgolsäuren bei einem Droge-Extrakt-Verhältnis von 35 bis 67 zu 1. Der in Tebonin® enthaltene Spezialextrakt EGb761® entspricht ebenfalls dieser Spezifikation.

In der Roten Liste (online Ausgabe 2007) findet man für den Wirkstoff Ginkgo 28 Einträge und insgesamt 54 verschiedene Produkte. Davon sind 34 blattextrakthaltige Monoprodukte und die übrigen 20 homöopathische Arzneimittel, die nach homöopathischen Regeln hergestellte Tinkturen oder Verdünnungen enthalten. Bei den Darreichungsformen der Ginkgoblätterextrakt-Produkte überwiegen die Filmtabletten (24), daneben gibt es noch ein Dragee und neun Produkte als Flüssigkeiten (Tropfen). Die Filmtabletten enthalten 40 mg, 50 mg, 60 mg, 80 mg oder 120 mg Extrakt aus Blättern von Ginkgo biloba.

Die Kommission E ist eine selbständige wissenschaftliche Kommission des ehemaligen deutschen Bundesgesundheitsamtes (BGA), heute Bundesinstitut für Arzneimittel und Medizinprodukte (BfArM). In den Jahren von 1980 bis 1994 bestand die Aufgabe der Kommission E darin, wissenschaftliches und erfahrungsheilkundliches Material zu erwünschten und unerwünschten Wirkungen pflanzlicher Drogen zusammenzutragen, auszuarbeiten und zu bewerten. Daraus entstanden die bis heute gültigen Monographien, die als Grundlage für die Neuzulassung und Nachzulassung pflanzlicher Arzneimittel gelten.

Die entsprechende Kommission E Monographie für Trockenextrakt aus Gingkobiloba-Blättern, veröffentlicht im Bundesanzeiger Nr. 133 vom 19.07.1994, gibt zur Behandlung von dementiellen Syndromen vor, dass täglich insgesamt 120 - 240 mg Trockenextrakt in zwei oder drei Einzeldosen eingenommen werden sollen. Diese Dosierungsempfehlung wird bei allen Produkten eingehalten, mit Ausnahme von Gingium 120 intens, von dem 1 - 2 mal täglich eine Filmtablette mit 120 mg Ginkgoextrakt eingenommen werden soll.

Bei anderen Indikationen als dem dementiellen Syndrom werden meist niedrigere Dosierungen empfohlen.

Popa A. beschreibt in "Ginkgo Biloba and Memory" (Pharmacotherapy Update, Vol V., No. V, September/October 2002) die chemische Zusammensetzung, die Wirkstoffe, die pharmakologische Wirkung, die Nebenwirkungen und die Dosierung von Ginkgo biloba Blätterextrakte. Außerdem werden Studien, die die Verwendung von Ginkgo biloba für die Gedächtnisverbesserung evaluieren, beschrieben.

Balch P. A. beschreibt in "Alzheimer's Disease" in "Prescription for Herbal Healing" (2002, Avery, USA, Seiten 183-186) die Verwendung verschiedener pflanzlichen Arzneimittel, wie Ginkgo biloba Extrakte, in der Behandlung der Alzheimer Krankheit.

Kanowski S. and Hoerr R. beschreiben in "Ginkgo biloba Extract EGb 761® in Dementia: Intent-to-treat Analyses of a 24-week, Multi-center, Double-blind, Placebo-controlled, Randomized Trial" (Pharmacopsychiatry 2003, 36: 297-303) eine klinische Studie in dem der Extrakt EGb 761® in verschiedenen Demenzerkrankungen getestet wurde.

Ein großes Problem bei der medikamentösen Behandlung von Krankheiten ist die mangelhafte Compliance (= Therapietreue) bei der Einnahme von Arzneimitteln. So ist bekannt, dass die Zuverlässigkeit der Einnahme mit der Anzahl der Tagesdosen abnimmt. Ein Ziel bei der Entwicklung von Arzneimitteln ist somit immer eine möglichst geringe Anzahl von Tagesdosen, wobei die einmal tägliche Gabe zur gleichen Uhrzeit die optimale Compliance zur Folge hat. Umgekehrt besteht bei mehrmaliger täglicher Einnahme die Gefahr, dass einzelne Einnahmen vergessen werden, was eine verminderte Wirksamkeit des Arzneimittels zur Folge hat. Dies gilt in besonderem Maße für die Behandlung des dementiellen Syndroms, das durch Gedächtnisstörungen, Konzentrationsstörungen, verminderte Alltagstauglichkeit und dadurch bedingt eine deutlich reduzierte Lebensqualität gekennzeichnet ist.

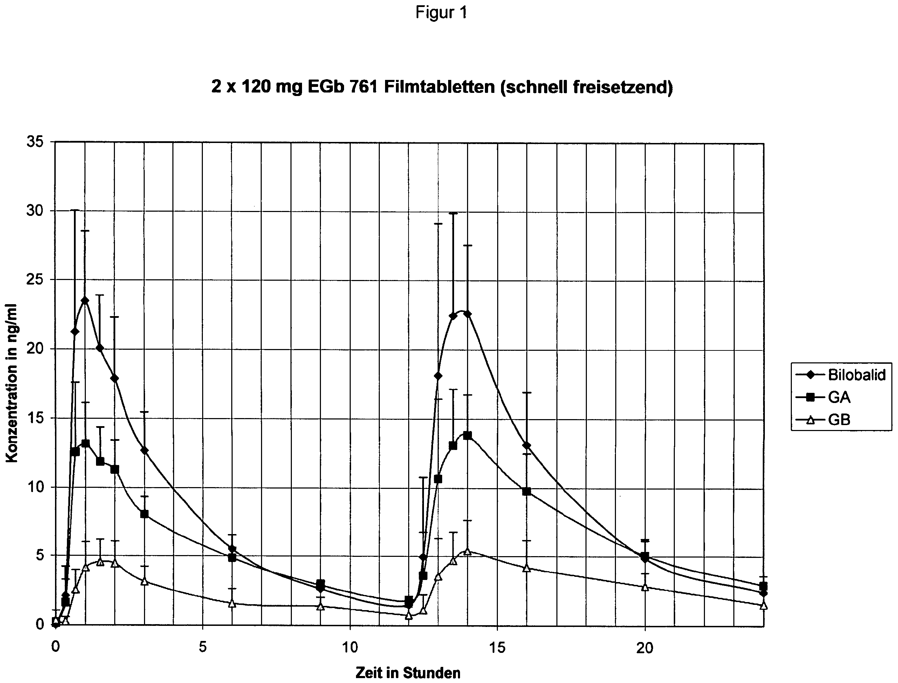

Dem Ziel der Entwicklung von Arzneimitteln zur einmal täglichen Einnahme steht das pharmakokinetische Verhalten vieler Wirkstoffe entgegen, die nach der Aufnahme in den Blutkreislauf nur eine kurze Körperverweilzeit bzw. kurze Eliminationshalbwertszeiten aufweisen. Dies erfordert die mehrmals tägliche Gabe zur Aufrechterhaltung einer wirksamen Wirkstoffkonzentration im Körper. Dies ist auch bei Ginkgoblätter-Extrakt der Fall, bei dem entsprechende Untersuchungen (Figur 1) zeigen, dass nach 12 Stunden nur noch sehr geringe Konzentrationen der wirksamkeitsmitbestimmenden Leitsubstanzen Bilobalid, Ginkgolid A (GA) und Ginkgolid B (GB) gemessen werden können und eine erneute Einnahme erforderlich wird.

Figur 1 zeigt die Konzentrationen von Bilobalid, Ginkgolid A (GA) und Ginkgolid B (GB) im Blutplasma von Menschen in Abhängigkeit von der Zeit, wobei zu Beginn und nach 12 Stunden jeweils eine Tablette mit 120 mg Ginkgo-Extrakt eingenommen wurde. Bei den angegebenen Werten handelt es sich um Mittelwerte aus 12 Probanden. Bei der verabreichten Menge handelt es sich um die gemäß Monographie empfohlene Tageshöchstdosis.

Die Aufgabe der vorliegenden Erfindung liegt somit in der Verbesserung der Behandlung des dementiellen Syndroms mit Ginkgo-Extrakten.

Obwohl dies aufgrund der kurzen Halbwertszeit keinesfalls zu erwarten war, wurde nun völlig überraschend gefunden, dass wichtige Parameter (wie "Verbesserung der Lebensqualität" und "für unbeteiligte Dritte erkennbare globale Verbesserung"), die zur Beurteilung der Wirksamkeit von Antidementiva gemessen werden und die in besonderem Maße als Indikatoren für die klinische Relevanz von Behandlungseffekten gelten, bei der einmal täglichen Gabe einer 240 mg Tablette stärker verbessert werden als bei Gabe von zweimal täglich jeweils einer 120 mg Tablette. Wie den Ausführungsbeispielen zu entnehmen ist, konnten mit der Filmtablette zu 240 mg EGb 761® bei täglicher Einmalgabe Wirkungen erzielt werden, die mit anderen Darreichungsformen bei Aufteilung der Tagesdosis in zwei Einzeldosen von 120 mg nicht nachweisbar waren. Damit ist belegt, dass die Filmtablette zu 240 mg EGb 761® bei täglicher oraler Einmalgabe einen über den der anderen Darreichungsformen hinausgehenden, eigenständigen therapeutischen Nutzen hat.

Die vorliegende Erfindung betrifft einen Extrakt aus Blättern von Ginkgo biloba als Mittel zur Verwendung zur Behandlung und Vorbeugung des dementiellen Syndroms sowie dessen Frühformen und Vorstufen, wobei einmal täglich 220 bis 260 mg und besonders bevorzugt 240 mg Ginkgo-Extrakt verabreicht werden. Das Mittel kann dabei ein Arzneimittel oder ein Lebensmittel wie z. B. ein Nahrungsergänzungsmittel (dietary supplement), ein funktionelles Lebensmittel (functional food, medical food), ein diätetisches Lebensmittel oder ein neuartiges Lebensmittel (novel food) sein.

Gegenstand der Erfindung ist des Weiteren die Verwendung eines Extraktes aus Blättern von Ginkgo biloba in Form eines Lebensmittels wie z. B. eines Nahrungsergänzungsmittels, eines funktionellen Lebensmittels, eines diätetischen Lebensmittels oder eines neuartigen Lebensmittels zur Behandlung und Vorbeugung des dementiellen Syndroms sowie dessen Frühformen und Vorstufen, wobei einmal täglich 220 bis 260 mg und besonders bevorzugt 240 mg Ginkgo-Extrakt verabreicht werden.

Unter einem dementiellen Syndrom (synonym auch als Demenz bezeichnet) versteht man eine erworbene Beeinträchtigung von Gedächtnis und einer oder mehrerer weiterer kognitiver Funktionen in einem Ausmaß, dass die Aktivitäten des täglichen Lebens und/oder die sozialen Beziehungen dadurch erheblich beeinträchtigt sind. Außer zu Gedächtnisstörungen führt das dementielle Syndrom auch zu Symptomen wie Konzentrationsstörungen, depressive Verstimmung, Schwindel, Ohrensausen und Kopfschmerzen. Als Ursachen für ein dementielles Syndrom kommen vor allem eine primäre Neurodegeneration bei Alzheimer-Krankheit und vaskuläre Ursachen (Störungen der Gehirndurchblutung) in Betracht. Die häufigsten Demenzformen sind demnach die Alzheimer-Demenz (auch als primär degenerative Demenz vom Alzheimer Typ bezeichnet), die vaskuläre Demenz (z. B. Multi-Infarkt-Demenz) und Mischformen von beiden. Durch sensitive kognitive Tests lassen sich heute auch bereits Frühformen und Vorstufen der Demenz erkennen, bei denen die international anerkannten diagnostischen Kriterien für die Demenz noch nicht erfüllt sind, aber bereits erkennbare kognitive Leistungseinbußen vorliegen. Man spricht deshalb von leichter kognitiver Beeinträchtigung (englisch: mild cognitive impairment, MCI) oder kognitiver Beeinträchtigung, die noch keine Demenz darstellt (englisch: cognitive impairment - no dementia, CIND). Alternsbezogene Symptomenkomplexe, die Vorstufen eines dementiellen Syndroms darstellen können, umfassen unterschiedliche Kombinationen aus zweien oder mehreren der folgenden Symptome: subjektiv empfundene Gedächtnisbeeinträchtigung, subjektive Beeinträchtigung anderer kognitiver Leistungskomponenten (z. B. Aufmerksamkeit, Konzentration, verbale Ausdrucksfähigkeit, Fähigkeit und Geschwindigkeit der Problemlösung, räumliches Vorstellungsvermögen, Planung und konzeptionelles Denken, Ausführung komplexer Tätigkeiten etc.), objektiv beeinträchtigte Gedächtnisleistung, objektive Beeinträchtigung anderer kognitiver Leistungskomponenten (z. B. Aufmerksamkeit, Konzentration, verbale Ausdrucksfähigkeit, Fähigkeit und Geschwindigkeit der Problemlösung, räumliches Vorstellungsvermögen, Planung und konzeptionelles Denken, Ausführung komplexer Tätigkeiten etc.), depressive Verstimmung, Angst, ängstliche Gestimmtheit, Apathie, Antriebsmangel, Gleichgültigkeit, Reizbarkeit, Erregung, Schlafstörungen, Störungen im Tag-Nacht-Rhythmus.

Bevorzugt sind Ginkgo-Extrakte entsprechend den Vorgaben des DAB 2003 und des noch nicht gültigen, aber bereits publizierten europäischen Arzneibuchs 6.1. Ginkgo-Extrakte gemäß DAB 2003 sind unter Verwendung von Aceton 60 % (m/m) und eines mehrstufigen Extraktionsverfahrens hergestellt, wobei das Verhältnis von Droge zu Extrakt (DEV) 35 bis 67 zu 1 beträgt und sind durch Gehalte an Flavonoiden von mindestens 22,0 % und höchstens 27,0 %, an Terpenlaktonen von mindestens 5,0 % und höchstens 7,0 % (davon 2,8 bis 3,4 % Ginkgolide A, B und C und 2,6 bis 3,2 % Bilobalid) und an Ginkgolsäuren von höchstens 5 ppm gekennzeichnet. Ginkgo-Extrakte gemäß Europäischem Arzneibuch 6.1 sind unter Verwendung von organischen Lösungsmitteln und deren Gemischen mit Wasser hergestellt, wobei das Verhältnis von Droge zu Extrakt (DEV) nicht näher angegeben ist und sind durch Gehalte an Flavonoiden von 22,0 % bis 27,0 %, an Bilobalid von 2,6 bis 3,2 % Bilobalid, an Ginkgoliden A, B und C von 2,8 bis 3,4 % und an Ginkgolsäuren von höchstens 5 ppm gekennzeichnet. Besonders bevorzugt ist der Ginkgo-Extrakt mit der Bezeichnung EGb® 761, der entsprechend dem DAB 2003 unter Verwendung von Aceton 60 % (m/m) und eines mehrstufigen Extraktionsverfahrens hergestellt ist, dessen Verhältnis von Droge zu Extrakt (DEV) 35 bis 67 zu 1 beträgt und der durch Gehalte an Flavonoiden von mindestens 22,0 % und höchstens 27,0 %, an Terpenlaktonen von mindestens 5,0 % und höchstens 7,0 % (davon 2,8 bis 3,4 % Ginkgolide A, B und C und 2,6 bis 3,2 % Bilobalid) und an Ginkgolsäuren von höchstens 5 ppm gekennzeichnet ist. Mit den genannten Eigenschaften entspricht EGb® 761 auch dem Europäischen Arzneibuch 6.1. Auch wenn dies im DAB 2003 und im Europäischen Arzneibuch 6.1 nicht explizit angegeben ist, handelt es sich bei allen vorstehenden Prozentangaben um Gew.-%. Besonders bevorzugt sind auch Ginkgo-Extrakte entsprechend WO2006/117169, die darüber hinaus einen verminderten Gehalt an Biflavonen und/oder 4'-O-Methylpyridoxin (< 20 ppm, bevorzugt < 10 ppm, besonders bevorzugt < 2 ppm) besitzen.
Die Herstellung des besonders bevorzugten Ginkgo-Extraktes EGb® 761 kann z. B. nach dem in EP431535B1 allgemein beschriebenen bzw. nach dem gemäß WO2006/117170 weiter optimierten Herstellungsverfahren erfolgen. Hierzu werden
(a) getrocknete und zerkleinerte Blätter von Ginkgo biloba bei einer Temperatur von etwa 40 bis 60 °C mit wässrigem Aceton (ca. 60 Gew.-% Aceton) extrahiert,
(b) das Aceton bis zu einem maximalen Gehalt von 5 Gew.-% abgetrennt,
(c) die verbliebene eingeengte wässrige Lösung mit Wasser bis zu einem Feststoffgehalt von ca. 10 Gew.-% verdünnt, bis auf eine Temperatur von höchstens 12 °C gekühlt und der resultierende Niederschlag entfernt,
(d) Ammoniumsulfat (ca. 30 Gew.-%) zur verbliebenen wässrigen Lösung zugefügt und die entstandene Lösung mit Methylethylketon oder einem Gemisch aus Methylethylketon und Aceton (6/4) extrahiert,
(e) die erhaltene organische Phase eingeengt und das so erhaltene Konzentrat mit Wasser und Ethanol so verdünnt, daß eine Lösung erhalten wird, die ca. 50 Gew.-% Wasser und 50 Gew.-% Ethanol bei einem Feststoffgehalt von ca. 10 Gew.-% enthält,
(f) eine wässrige Lösung von Bleihydroxidacetat zu der auf diese Weise erhaltenen Lösung zugefügt und der entstandene Niederschlag entfernt,
(g) die verbliebene wässrig-ethanolische Lösung mit Heptan extrahiert, um die Alkylphenolverbindungen weiter zu entfernen,
(h) die verbliebene wässrig-ethanolische Lösung unter vermindertem Druck eingeengt und ca. 20 Gew.-% Ammoniumsulfat zugefügt,
(i) die erhaltene Lösung mit einem Gemisch aus Methylethylketon und Ethanol (6/4) extrahiert,
(k) die resultierende organische Phase mit max. 20 Gew.-% Ammoniumsulfat getrocknet und eingeengt, so mit Ethanol versetzt, dass ein Ethanol-Gehalt von mindestens 80 Gew.-% resultiert, und 2 bis 10 h bei ≤ 12 °C gehalten und filtriert,
(l) das resultierende Filtrat unter vermindertem Druck eingeengt und getrocknet, wobei ein Trockenextrakt mit einem Wassergehalt von weniger als 5 % erhalten wird.

Zur Herstellung der besonders bevorzugten Ginkgo-Extrakte entsprechend WO2006/117169 mit einem verminderten Gehalt an Biflavonen und/oder 4'-O-Methylpyridoxin wird z. B. die Lösung aus obigem Schritt (k) gegebenenfalls mit wässrigem Ethanol verdünnt, über ein Adsorberharz und/oder einen Ionenaustauscher filtriert, wobei die zu entfernenden Stoffe auf dem Harz zurückgehalten werden, und unter vermindertem Druck zu einem Trockenextrakt mit einem Wassergehalt von weniger als 5 % getrocknet. Die Abreicherung von Biflavonen und/oder 4'-O-Methylpyridoxin führt dabei nicht zu einer signifikanten Beeinflussung der Gehalte an wirksamkeitsbestimmenden Komponenten.

Die erfindungsgemäß verwendbaren Ginkgo-Extrakte können in Form von Pulvern, Granulaten, Brausezubereitungen, Tabletten, Dragees, Kapseln oder Flüssigkeiten vorzugsweise oral verabreicht werden. Zur Herstellung von Tabletten wird der Extrakt mit geeigneten pharmazeutisch verträglichen Hilfsstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxypropylmethylcellulose, Polyethylenglykol, Farbstoffen (z. B. Titandioxid), und Talkum versehen werden. Der erfindungsgemäß verwendete Extrakt kann auch, gegebenenfalls unter Zusatz von Hilfsstoffen wie z. B. Füllmittel, Fließregulierungsmittel etc., in Kapseln abgefüllt werden.
Bevorzugte Darreichungsform im Fall der Tabletten sind Filmtabletten. Ferner handelt es sich bei den bevorzugten Darreichungsformen um Formen, die den Extrakt im Körper schnell freisetzen und die im Europäischen Arzneibuch 6.0 als "Conventional-release dosage forms" bezeichnet werden.

Ausführungsformen der vorliegenden Erfindung sind Folgende:
Extrakt aus Ginkgo biloba als Mittel zur Verwendung zur Behandlung und Vorbeugung des dementiellen Syndroms sowie dessen Frühformen und Vorstufen, wobei einmal täglich 220 bis 260 mg Ginkgo-Extrakt, vorzugsweise oral, verabreicht werden, wobei der Extrakt 22,0 bis 27,0 Gew.-% Flavonoide und 2,6 bis 3,2 Gew.-% Bilobalid und 2,8 bis 3,4 Gew.-% Ginkgolide A, B und C (in der Summe) enthält. Der Extrakt enthält zudem höchstens 5 ppm Ginkgolsäuren. Des Weiteren enthält der Extrakt vorzugsweise weniger als 20 ppm, besonders bevorzugt weniger als 10 ppm und insbesondere weniger als 2 ppm 4'-O-Methylpyridoxin.
Der Extrakt der vorstehend angegebenen Zusammensetzung wird einmal täglich oral in einer Menge von 220 bis 260 mg und insbesondere in einer Menge von 240 mg verabreicht.
Eine ganz besonders bevorzugte Ausführungsform der vorliegenden Erfindung ist der Extrakt aus Ginkgo biloba als Mittel zur Verwendung zur Behandlung und Vorbeugung des dementiellen Syndroms sowie dessen Frühformen und Vorstufen, wobei einmal täglich oral 240 mg Ginkgo-Extrakt verabreicht werden, wobei es sich bei dem Ginkgo-Extrakt um den an sich bekannten Extrakt EGb 761® handelt. Bei dem dementiellen Syndrom handelt es sich hierbei vorzugsweise um leichte Demenz, mittelschwere Demenz oder leichte bis mittelschwere Demenz.

### Beispiele

Bei dem nachstehend als EGb 761® bezeichneten Extrakt handelt es sich um einen Spezialextrakt der Fa. Dr. Willmar Schwabe GmbH & Co. KG aus Blättern von Ginkgo biloba entsprechend den Vorgaben des Deutschen Arzneibuchs (DAB) mit Gehalten an Flavonoiden von 22,0 bis 27,0 Gew.-%, an Terpenlaktonen von 5,0 bis 7,0 Gew.-% (davon 2,8 bis 3,4 Gew.-% Ginkgolide A, B und C und 2,6 bis 3,2 Gew.-% Bilobalid) und an Ginkgolsäuren von höchstens 5 ppm. Die Gehaltsbestimmungen erfolgen dabei gemäß DAB, wobei die Flavonoide nach saurer Hydrolyse als Quercetin, Kämpferol und Isorhamnetin bestimmt und als Flavonoidglycoside berechnet werden.

### Vergleichsbeispiel 1 - Verbesserung der Lebensqualität

In einer randomisierten, placebokontrollierten Doppelblindstudie mit EGb 761® an Patienten mit dementiellem Syndrom (leichte bis mittelschwere Demenz) [Schneider et al. 2005] wurde die Lebensqualität der Patienten untersucht. In dieser Studie wurde EGb 761® in Form von Filmtabletten zu 60 mg bzw. 120 mg eingesetzt, jeweils 2 Tabletten täglich zum Erzielen von Tagesdosen von 120 mg bzw. 240 mg. Unter diesem Behandlungsregime konnte für keine der geprüften Dosierungen von EGb 761® unter Anwendung der Progressive Deterioration Scale (PDS) [DeJong et al. 1989], einer speziell für Demenzpatienten entwickelten und validierten Lebensqualitätsskala, im Vergleich zu Placebo ein signifikanter Effekt auf die Lebensqualität gezeigt werden (p = 0.7).

### Erfindungsgemäßes Beispiel 1 - Verbesserung der Lebensqualität

Durch Behandlung von Patienten mit dementiellem Syndrom (leichte bis mittelschwere Demenz) mit der schnell freisetzenden Filmtablette zu 240 mg EGb 761® (einmal täglich) konnte zum ersten mal eine Verbesserung der Lebensqualität der Patienten unter Behandlung mit EGb 761® in einer randomisierten, placebokontrollierten Doppelblindstudie nachgewiesen werden. Unter Behandlung mit der Filmtablette zu 240 mg EGb 761® in einer Dosierung von einer Tablette täglich war im Verlauf der 24-wöchigen randomisierten Behandlung eine Verbesserung der Lebensqualität zu beobachten, die sich in einer mittleren Zunahme des Gesamtwertes in der speziell für Demenzpatienten entwickelten und validierten Lebensqualitätsskala DEMQOL-Proxy [Smith et al. 2005] um 3,38 (s = 8,45) Punkte äußerte, während in der Placebogruppe nur eine deutlich geringere Verbesserung um 1,36 (s = 6,62) Punkte zu sehen war. Der Unterschied zwischen den beiden Behandlungsgruppen war mit p = 0.004 statistisch signifikant. Bei Betrachtung des Verlaufs über die 24-wöchige Behandlungsdauer erkennt man, dass der mittlere Behandlungseffekt unter EGb 761® (1 x 240 mg) kontinuierlich zunimmt, während es in der Placebogruppe nur zu einer vorübergehenden leichten Verbesserung kommt, wie sie durch die vermehrte Zuwendung im Rahmen einer klinischen Prüfung häufig zu beobachten ist, die aber infolge der naturgemäßen Krankheitsprogression noch während des Beobachtungszeitraums weitgehend wieder verloren geht [Walach et al. 2005]. Betrachtet man die Ergebnisse auf Patientenebene, so erkennt man nach 24-wöchiger Behandlung Verbesserungen der Lebensqualität bei 113 (55,9 %) der mit EGb 761® (1 x 240 mg) behandelten Patienten, hingegen nur bei 90 (44,6 %) der Patienten, die Placebo erhielten (p < 0.05).

Unter Behandlung mit der Filmtablette mit 240 mg EGb 761® als tägliche Einmalgabe konnte zum ersten mal in einer randomisierten, kontrollierten klinischen Prüfung eine im Vergleich zur Placebogabe signifikante Verbesserung der Lebensqualität von Patienten mit dementiellem Syndrom (leichte bis mittelschwere Demenz) nachgewiesen werden. Darüber hinaus zeigte sich eine deutliche Verbesserung der Lebensqualität gegenüber der Einnahme von 2 x 60 mg bzw. 2 x 120 mg EGb 761® (Vergleichsbeispiel 1).

### Vergleichsbeispiel 2 - Für unbeteiligte Dritte erkennbare globale Verbesserung

In einer randomisierten, placebokontrollierten Doppelblindstudie mit EGb 761® an Patienten mit demetiellem Syndrom (leichte bis mittelschwere Demenz) [Schneider et al. 2005] wurde ebenfalls die globale Zustandsveränderung der Patienten durch unabhängige, unbeteiligte Dritte anhand der von der Alzheimer's Disease Cooperative Study entwickelten und validierten Clinical Global Impression of Change Skala (ADCS-CGIC) beurteilt. In dieser Studie war kein statistisch signifikanter Vorteil einer Behandlung mit EGb 761® in den Dosierungen von 2 x 60 mg bzw. 2 x 120 mg pro Tag zu erkennen (p = 0.2).

### Erfindungsgemäßes Beispiel 2 - Für unbeteiligte Dritte erkennbare globale Verbesserung

Durch Behandlung mit der schnell freisetzenden Filmtablette zu 240 mg (einmal täglich) konnte zum ersten mal bei Patienten mit demetiellem Syndrom (leichte bis mittelschwere Demenz) eine globale Zustandsverbesserung solchen Ausmaßes erzielt werden, dass sie für unabhängige Beurteiler, die weder an der Behandlung des Patienten beteiligt waren noch irgendwelche Untersuchungsbefunde kannten, im Gespräch mit den Patienten und den Angehörigen eindeutig zu erkennen war. Die Beurteilung durch die unabhängigen, unbeteiligten Dritten erfolgte ebenfalls anhand der von der Alzheimer's Disease Cooperative Study entwickelten und validierten Clinical Global Impression of Change Skala (ADCS-CGIC) [Schneider et al. 1997]. Diese Skala umfasst sieben Kategorien von "ausgeprägte Verbesserung" (Skalenwert 1) über "keine Veränderung" (Skalenwert 4) bis "ausgeprägte Verschlechterung" (Skalenwert 7). Unter Behandlung mit EGb 761® (1 x 240 mg) war bei 109 Patienten (54,0 %) eine globale Zustandsverbesserung festzustellen, jedoch nur bei 52 Patienten (25,7 %) der Patienten, die Placebo erhielten (p < 0.0001). Zieht man in Betracht, dass sich Patienten mit Demenz im Lauf der Zeit naturgemäß verschlechtern und daher bereits eine ausbleibende Verschlechterung als Behandlungserfolg gewertet werden kann, so finden sich unter den mit EGb 761® (1 x 240 mg) behandelten Patienten 186 (92,1 %) und in der Placebogruppe 135 Patienten (66,8 %), bei denen während der 24-wöchigen Behandlungszeit eine Verschlechterung ausblieb (p < 0.0001).

Unter Behandlung mit der Filmtablette mit 240 mg EGb 761® als tägliche Einmalgabe konnte zum ersten mal in einer randomisierten, kontrollierten klinischen Prüfung eine im Vegleich zu Placebo signifikant bessere Beurteilung der globalen Zustandsänderung durch unabhängige, nicht an der Behandlung der Patienten beteiligte und nicht über die Befunde der Patienten informierte Dritte gezeigt werden. Darüber hinaus zeigte sich eine deutliche Verbesserung der globalen Zustandsänderung gegenüber der Einnahme von 2 x 60 mg bzw. 2 x 120 mg EGb 761® (Vergleichsbeispiel 2).

### Erfindungsgemäßes Beispiel 3 - 240 mg Tablette zur einmal täglichen peroralen Einnahme ("Conventional-release dosage form" gemäß Europäischem Arzneibuch 6.0)

| Bestandteil | Mengenanteil in mg pro Tablette |
|---|---|
| Ginkgoblätter-Extrakt EGb ® 761 | 240,0 |
| Laktose Monohydrat | 160,0 |
| Mikrokristalline Cellulose | 290,0 |
| Maisstärke | 50,0 |
| Hochdisperses Siliciumdioxid | 10,0 |
| Croscarmellose-Natrium | 40,0 |
| Magnesiumstearat | 10,0 |
| Hypromellose | 20,0 |
| Macrogol | 5,0 |
| Talkum | 2,5 |
| Titandioxid | 1,0 |

Der Ginkgoblätter-Extrakt wird mit der Laktose (Füllmittel), der mikrokristallinen Zellulose (Füll-/Bindemittel), der Maisstärke (Bindemittel), der Crosscarmellose (Zerfallsbeschleuniger) und dem hochdispersen Siliciumdioxid (Fließregulierungsmittel) in einem Arbeitsgang in einem geeigneten Mischer gemischt. Das Magnesiumstearat (Schmiermittel) wird zugefügt und erneut kurz gemischt. Diese Mischung wird auf einer Rundlauftablettenpresse zu ovalen, gewölbten Tabletten mit einer mittleren Masse von 800 mg, einer Länge von 17 mm und einer Breite von 8 mm verpresst.

Hypromellose (Überzugsmittel) und Macrogol (Weichmacher) werden in gereinigtem Wasser unter Rühren gelöst und Talkum (Antiklebemittel) sowie Titandoxid (Farbpigment) hinzugefügt und durch Rühren dispergiert. Die Mischung wird zur Quellung und vollständigen Auflösung des Polymers 12 Stunden stehen gelassen und anschließend in einem Trommelcoater auf die Tabletten aufgesprüht. Das Endprodukt sind weiß gefärbte Filmtabletten mit je 240 mg Ginkgoblätter-Extrakt zur peroralen Einnahme.

### Referenzen

DeJong R, Osterlund OW, Roy GW. Measurement of Quality-of-Life changes in patients with Alzheimer's disease. Clinical Therapeutics 1989;11:545-554.
Schneider LS, Olin JT, Doody RS, Clark CM, Morris JC, Reisberg B, Schmitt FA, Grundman M, Thomas RG, Ferris SH, and the Alzheimer's Disease Cooperative Study. Validity and Reliability of the Alzheimer's Disease Cooperative Study - Clinical Global Impression of Change. Alzheimer Disease and Associated Disorders 1997;11(suppl 2):S22-S32.
Schneider LS, DeKosky ST, Farlow MR, Tariot PN, Hoerr R, Kieser M. A randomized, double-blind, placebo-controlled trial of two doses of Ginkgo biloba extract in dementia of the Alzheimer's type. Current Alzheimer Research 2005;2:541-551.
Smith SC, Lamping DL, Banerjee S, Harwood R, Foley B, Smith P, Cook JC, Murray J, Prince M, Levin E, Mann A, Knapp M. Measurement of health-related quality of life for people with dementia: development of anew instrument (DEMQOL) and an evaluation of current methodology. Health Technology Assessment 2005;9(10).
Walach H, Sadaghiani C, Dehm C, Bierman D. The therapeutic effect of clinical trials: understanding placebo response rates in clinical trials - A secondary analysis. BMC Medical Research Methodology 2005;5:26.

## Patentansprüche

1. Extrakt aus Blättern von Ginkgo biloba als Mittel zur Verwendung zur Behandlung und Vorbeugung des dementiellen Syndroms sowie dessen Frühformen und Vorstufen, wobei einmal täglich 220 bis 260 mg Extrakt verabreicht werden und wobei der Extrakt 22,0 bis 27,0 Gew.-% Flavonoide und 2,6 bis 3,2 Gew.-% Bilobalid und 2,8 bis 3,4 Gew.-% Ginkgolide A, B und C (in der Summe) und höchstens 5 ppm Ginkgolsäuren enthält.

2. Extrakt zur Verwendung nach Anspruch 1, wobei das dementielle Syndrom ausgewählt ist aus leichter Demenz, mittelschwerer Demenz, Alzheimer-Demenz, vaskulärer Demenz und deren Mischformen.

3. Extrakt zur Verwendung nach Anspruch 1 oder 2, wobei es sich bei den Frühformen und Vorstufen der Demenz um leichte kognitive Beeinträchtigungen (MCI), kognitive Beeinträchtigungen, die noch keine Demenz darstellen (CIND), subjektiv empfundene Gedächtnisbeeinträchtigungen, subjektive Beeinträchtigungen anderer kognitiver Leistungskomponenten, objektiv beeinträchtigte Gedächtnisleistungen, objektive Beeinträchtigungen anderer kognitiver Leistungskomponenten, depressive Verstimmung, Angst, ängstliche Gestimmtheit, Apathie, Antriebsmangel, Gleichgültigkeit, Reizbarkeit, Erregung, Schlafstörungen oder Störungen im Tag-Nacht-Rhythmus handelt.

4. Extrakt zur Verwendung nach Anspruch 3, wobei die anderen subjektiv und/oder objektiv beeinträchtigten kognitiven Leistungskomponenten ausgewählt sind aus Aufmerksamkeit, Konzentration, verbaler Ausdrucksfähigkeit, Fähigkeit und Geschwindigkeit der Problemlösung, räumlichem Vorstellungsvermögen, Planung und konzeptionellem Denken, Ausführung komplexer Tätigkeiten.

5. Extrakt zur Verwendung nach einem der Ansprüche 1 bis 4, wobei der Extrakt weniger als 20 ppm 4'-O-Methylpyridoxin enthält.

6. Extrakt zur Verwendung nach Anspruch 5, wobei der Extrakt weniger als 10 ppm 4'-O-Methylpyridoxin enthält.

7. Extrakt zur Verwendung nach Anspruch 5, wobei der Extrakt weniger als 2 ppm 4'-O-Methylpyridoxin enthält.

8. Extrakt zur Verwendung nach einem der Ansprüche 1 bis 7, wobei der Extrakt in Form von Pulvern, Granulaten, Brausezubereitungen, Tabletten, Dragees, Kapseln oder Flüssigkeiten verabreicht wird.

9. Extrakt zur Verwendung nach Anspruch 8, wobei die Verabreichung oral erfolgt.

10. Extrakt zur Verwendung nach Anspruch 8 oder 9, wobei die Tablette eine Filmtablette ist.

11. Extrakt zur Verwendung nach einem der Ansprüche 8 bis 10, wobei es sich um eine Tablette mit schneller Wirkstofffreisetzung handelt.

12. Extrakt zur Verwendung nach einem der Ansprüche 1 bis 11, wobei einmal täglich 240 mg Ginkgo-Extrakt verabreicht werden.

13. Extrakt zur Verwendung nach einem der Ansprüche 1 bis 12, wobei einmal täglich der Extrakt EGb 761® in Form eines Mittels oral zur Behandlung von leichter, mittelschwerer oder leichter bis mittelschwerer Demenz verabreicht wird.

14. Extrakt zur Verwendung nach einem der Ansprüche 1 bis 13, wobei das Mittel ein Arzneimittel ist.

15. Extrakt zur Verwendung nach einem der Ansprüche 1 bis 13, wobei das Mittel ein Lebensmittel ist.

16. Extrakt zur Verwendung nach Anspruch 15, wobei das Lebensmittel ein Nahrungsergänzungsmittel (dietary supplement), ein funktionelles Lebensmittel (functional food, medical food), ein diätetisches Lebensmittel oder ein neuartiges Lebensmittel (novel food) ist.

## Claims

1. Extract made of leaves of Ginkgo biloba as an agent for use for the treatment and prevention of the dementia syndrome and the early stages and pre-stages thereof, wherein 220 to 260 mg of extract is administered once per day and wherein the extract contains 22.0% to 27.0% by weight of flavonoids and 2.6% to 3.2% by weight of bilobalide, and 2.8% to 3.4% by weight of ginkgolides A, B and C (as sum) and at most 5 ppm of ginkgolic acids.

2. Extract for use according to claim 1, wherein the dementia syndrome is selected from light dementia, moderate dementia, Alzheimer dementia, vascular dementia and their mixed forms.

3. Extract for use according to claim 1 or 2, wherein the early stages and pre-stages of dementia are light cognitive impairments (MCI), cognitive impairments, which are not dementia yet (CIND), subjectively perceived impairments of the memory, subjective impairments of other cognitive components of performance, objectively impaired memory performance, objective impairments of other cognitive components of performance, depressive disorder, anxiety, frightened mood, apathy, lack of motivation, indifference, irritability, excitement, sleeping disorders, and disorders of day and night rhythm.

4. Extract for use according to claim 3, wherein the other subjectively and/or objectively impaired cognitive components of performance are selected from attention, concentration, ability of verbal expression, ability and velocity to solve problems, ability of spatial imagination, planning, conceptional thinking, and carrying out of complex activities.

5. Extract for use according to any one of claims 1 to 4, wherein the extract contains less than 20 ppm 4'-O-methyl pyridoxine.

6. Extract for use according to claim 5, wherein the extract contains less than 10 ppm 4'-O-methyl pyridoxine.

7. Extract for use according to claim 5, wherein the extract contains less than 2 ppm 4'-O-methyl pyridoxine.

8. Extract for use according to any one of claims 1 to 7, wherein the extract is administrated in form of powders, granulates, effervescent preparations, tablets, dragees, capsules, or liquids.

9. Extract for use according to claim 8, wherein the administration is oral.

10. Extract for use according to claim 8 or 9, wherein the tablet is a film-coated tablet.

11. Extract for use according to any one of claims 8 to 10, wherein the tablet is a rapid release tablet.

12. Extract for use according to any one of claims 1 to 11, wherein 240 mg of Ginkgo extract are administrated once per day.

13. Extract for use according to any one of claims 1 to 12, wherein the extract EGb 761® is administrated in form of an agent orally once per day to treat light, moderate or light to moderate dementia.

14. Extract for use according to any one of claims 1 to 13, wherein the agent is a drug.

15. Extract for use according to any one of claims 1 to 13, wherein the agent is a food product.

16. Extract for use according to claim 15, wherein the food product is a dietary supplement, a functional/medical food, a dietetic food or a novel food.

## Revendications

1. Extrait de feuilles de Gingko biloba en tant qu'agent pour une utilisation pour le traitement ou la prévention du syndrome de démence ainsi que de ses formes précoces et précurseurs, dans lequel, une fois par jour, 220 à 260 mg d'extrait sont administrés, et dans lequel ledit extrait contient 22,0 à 27,0% en poids de flavonoïdes et 2,6 à 3,2% en poids de biobalide et 2,8 à 3,4% en poids de ginkgolides A, B et C (au total) et au plus 5 ppm d'acides ginkgoliques.

2. Extrait pour l'utilisation selon la revendication 1, dans lequel le syndrome de démence est choisi parmi la démence légère, la démence modérée, la démence d'Alzheimer, la démence vasculaire et des formes mixtes de celles-ci.

3. Extrait pour l'utilisation selon la revendication 1 ou 2, dans lequel les formes précoces et précurseurs de la démence sont des déficiences légères (MCI), des déficiences cognitives, qui ne constituent pas encore la démence (CIND), la perception subjective des déficiences de la mémoire, des déficiences subjectives d'autres composants de performance cognitive, la déficience objective de la capacité de mémoire, la déficience objective d'autres composants de performance cognitive, l'humeur dépressive, l'anxiété, l'humeur anxieuse, l'apathie, le manque d'entrain, l'indifférence, l'irritabilité, l'excitation, des troubles du sommeil ou des troubles du rythme jour-nuit.

4. Extrait pour l'utilisation selon la revendication 3, dans lequel les autres composants de performance cognitive, qui sont perçues subjectivement et/ou objectivement comme troublés, sont choisis parmi l'attention, la concentration, les compétences en communication verbale, la capacité et la vitesse de la résolution de problèmes, l'imagination spatiale, la planification et la pensée conceptuelle et l'exécution d'activités complexes.

5. Extrait pour l'utilisation selon l'une des revendications 1 à 4, ledit extrait contenant moins de 20 ppm de 4'-O-méthylpyridoxine.

6. Extrait pour l'utilisation selon la revendication 5, ledit extrait contenant moins de 10 ppm de 4'-O-méthylpyridoxine.

7. Extrait pour l'utilisation selon la revendication 5, ledit extrait contenant moins de 2 ppm de 4'-O-méthylpyridoxine.

8. Extrait pour l'utilisation selon l'une des revendications 1 à 7, dans lequel l'extrait est administré sous forme de poudres, de granulés, de préparations effervescentes, de comprimés, de dragées, de capsules ou de liquides.

9. Extrait pour l'utilisation selon la revendication 8, dans lequel l'administration est par voie orale.

10. Extrait pour l'utilisation selon la revendication 8 ou 9, dans lequel le comprimé est un comprimé pelliculé.

11. Extrait pour l'utilisation selon l'une des revendications 8 à 10, dans lequel le comprimé est un comprimé à libération rapide.

12. Extrait pour l'utilisation selon l'une des revendications 1 à 11, dans lequel 240 mg d'extrait de ginkgo sont administrés une fois par jour.

13. Extrait pour l'utilisation selon l'une des revendications 1 à 12, dans lequel l'extrait EGb 761® est administré une fois par jour par voie orale sous forme d'un agent pour le traitement de la démence légère, modérée ou modérée à sévère.

14. Extrait pour l'utilisation selon l'une des revendications 1 à 13, dans lequel l'agent est un médicament.

15. Extrait pour l'utilisation selon l'une des revendications 1 à 13, dans lequel l'agent est un aliment.

16. Extrait pour l'utilisation selon la revendication 15, dans lequel l'aliment est un complément alimentaire (dietary supplément), un aliment fonctionnel (functional food, médical food), un aliment diététique ou un aliment nouveau (novel food).
